# EUROPEAN PATENT SPECIFICATION

(11) **EP 2 119 413 B1**
(45) Date of publication and mention of the grant of the patent: **13.07.2016**
(21) Application number: 07737788.5
(22) Date of filing: 05.03.2007
(51) Int. Cl.: A61B 90/00

(54) **MEDICAL SYSTEM AND MEDICAL DISPLAY DEVICE**
MEDIZINISCHES SYSTEM UND MEDIZINISCHE ANZEIGEVORRICHTUNG
SYSTÈME MÉDICAL ET DISPOSITIF D'AFFICHAGE MÉDICAL

(43) Date of publication of application: 18.11.2009
(73) Proprietor: Olympus Corporation, Shibuya-ku Tokyo (JP)
(72) Inventor: SEKIGUCHI, Kiyoshi, Tokyo 151-0072 (JP); ITO, Masaru, Tokyo 151-0072 (JP); OZAKI, Takashi, Tokyo 151-0072 (JP)
(74) Representative: von Hellfeld, Axel
(86) International application number: PCT/JP2007/054213
(87) International publication number: WO 2008/107964

(56) References cited:
- JP-A- 10 165 404
- JP-A- 11 184 567
- JP-A- 2003 043 978
- JP-A- 2003 043 978
- JP-A- 2005 080 195
- JP-A- 2006 012 262
- US-A1- 2005 043 615

## Description

### Technical Field

The present invention relates to a medical system equipped with a panel for displaying states of a plurality of medical devices and input operations.

### Background Art

A medical endoscope system equipped with an endoscope has been introduced as the medical system which employs a plurality of devices to be controlled.

A generally employed medical endoscope system includes devices to be controlled, for example, an endoscope used for observations, a camera head connected to the endoscope, a medical camera unit for processing an image signal shot by the camera head, a light source unit for supplying illuminating light to a subject and a monitor for displaying an image of the subject.

The medical staff inserts the endoscope into the body cavity and the like, irradiates the illuminating light to the subject from the light source unit to allow the endoscope to obtain an optical image of the subject. The image signal of the subject shot by the camera head is then subjected to a signal processing by a camera unit for endoscope such that the image of the subject is displayed on the monitor. The above-structured endoscope system is employed for observation and inspection of the body cavity and the like.

Under the recent surgical operation with the aid of the endoscope, in addition to the aforementioned instrument, the surgical equipment, for example, the pneumoperitoneum unit for expanding the abdominal cavity, the treatment device for the operative technique, and a high-frequency cauterization unit for excising the body tissue has been used for performing various treatments while observing the treatment site with the endoscope.

As the treatments will be performed by operating the aforementioned plural devices simultaneously, the medical system provided with the operation means for centrally operating the respective devices and the display means for centrally displaying the states of the respective devices so as to allow the medical staff to set the respective devices to be operated suitable for the treatment and to easily confirm the set states of the respective devices is disclosed in, for example, Japanese Patent Application Laid Open Publication No. 2003-175044.

The aforementioned related art discloses the structure where two trolleys are provided at both sides of the operating bed, each of which is provided with a plurality of devices and the central display panel for central display. One of the trolleys is equipped with a central operation panel. The plurality of devices, the central display panel and the central operation panel are connected to the system controller. In the related art, the system controller serves to centrally control the system so as to realize the environment which allows the surgical operation to be smoothly performed under the observation with the endoscope.

However, in a place with little storage or set space such as a narrow surgery room, when the central display panel and the central operation panel are separately provided as described above, the panels protrudingly occupy the space in the surgery room. As a result, the operator feels a sense of being oppressed, and it is difficult to improve the operability.

In addition, the central display panel and the central operation panel are separately provided, so that there has been a problem of an increase in the cost of the system.

JP 2003043978 (A) discloses a face-to-face type display device whose display contents of screens can be easily, surely and alternately viewed by two persons facing each other. Since vertical and horizontal directions of the display contents on the screen of a liquid crystal display unit are reversed when the liquid crystal display unit is moved to a position A from a position C, the display contents are normally displayed also to both of a visitor and a salesclerk.

The present invention has been achieved in view of above-described points, and it is an object of the present invention is to provide a medical system which can achieve cost reduction and can be placed in a narrow set space.

### Disclosure of Invention

### Means for Solving the Problem

The medical system according to the present invention comprises the features of claim 1.

The medical display apparatus not forming part of the present invention is provided with a panel portion which is connected to a control unit for controlling operations of a plurality of medical devices so as to be communicated therewith, and includes a display unit for displaying information with respect to the plurality of medical devices and a sensor unit for detecting an input operation, an information storage unit which stores graphical user interface information for display and graphical user interface information for operation, a detection unit for detecting whether the panel portion is disposed at least one predetermined position, and a display selection control unit which executes a control to select the information between the graphical user interface information for display and the graphical user interface information for operation which are stored in the information storage unit based on a detection signal of the detection unit so as to be displayed on the display unit.

### Brief Description of the Drawings

Fig. 1 is a view showing a general structure of the medical endoscope system according to Embodiment 1 of the present invention;
Fig. 2 is a block diagram showing main components of the structure shown in Fig. 1;
Fig. 3A is a view showing the trolley to which the panel PC is detachably mounted;
Fig. 3B is a view showing the state where the panel PC is attached to the
   panel PC attachment mount;
Fig. 4 is a block diagram showing the main components of the system controller and the panel PC including the inner structures;
Fig. 5 is a view showing an example of screens of the panel PC for displaying GUI for display and GUI for operation which will be selected in accordance with the detection results of the sensor;
Fig. 6 is a flowchart of a control routine for selecting the display screen;
Fig. 7 is a block diagram showing main components of the system controller and the touch panel including the inner structures according to Embodiment 2 of the present invention;
Fig. 8A is a front view showing a trolley to which the touch panel is movably fixed;
Fig. 8B is a side view of the touch panel shown in Fig. 8A;
Fig. 9 is a view showing an example of screens of the touch panel which displays the GUI for display and the GUI for operation which will be selected in accordance with the detection result of the sensor;
Fig. 10 is a flowchart of a control routine for selecting the display screen;
Fig. 11 is a block diagram showing main components of the system controller and the touch panel including the inner structures according to a modified example of Embodiment 2;
Fig. 12 is a flowchart of a control routine for selecting the display screen according to the modified example;
Fig. 13 is a block diagram showing main components of the system controller and the touch panel including the inner structures according to Embodiment 3;
Fig. 14 is a plan view schematically showing a touch panel holding mechanism for rotatably holding the touch panel on the upper surface of the trolley;
Fig. 15 is a front view schematically showing the touch panel holding mechanism when viewed from the front as shown in Fig. 14;

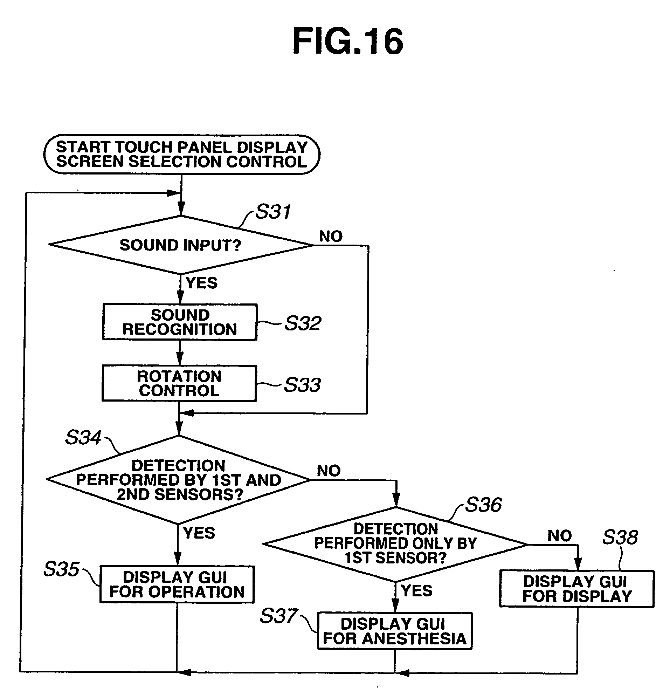

Fig. 16 is a flowchart of a control routine for selecting the display screen;
Fig. 17A is a side view showing a cross section of a part of the touch panel holding mechanism according to the modified example of Embodiment 3;
Fig. 17B is a side view partially showing the structure shown in Fig. 17A; and
Fig. 18 is a flowchart of a control routine for selecting the display screen according to the modified example.

### Best Mode for Carrying Out the Invention

Embodiments of the present invention will be described referring to the drawings.

### Embodiment 1

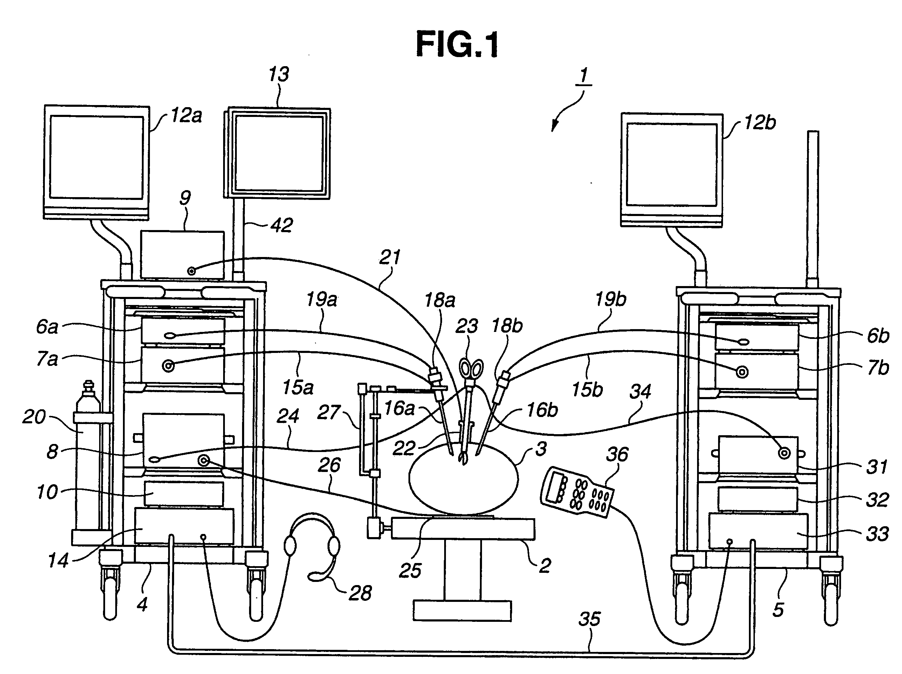

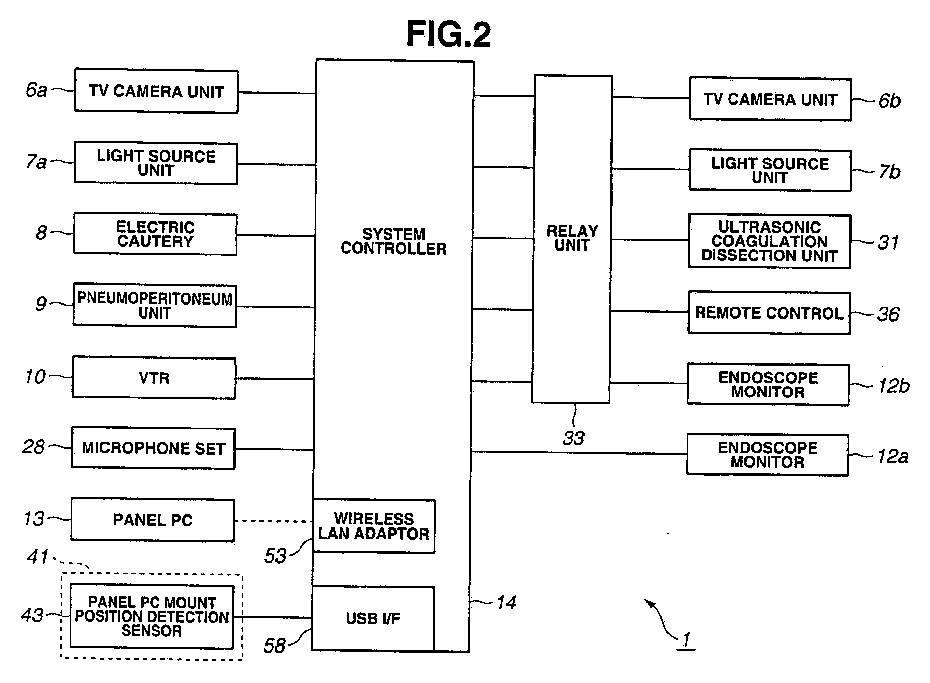

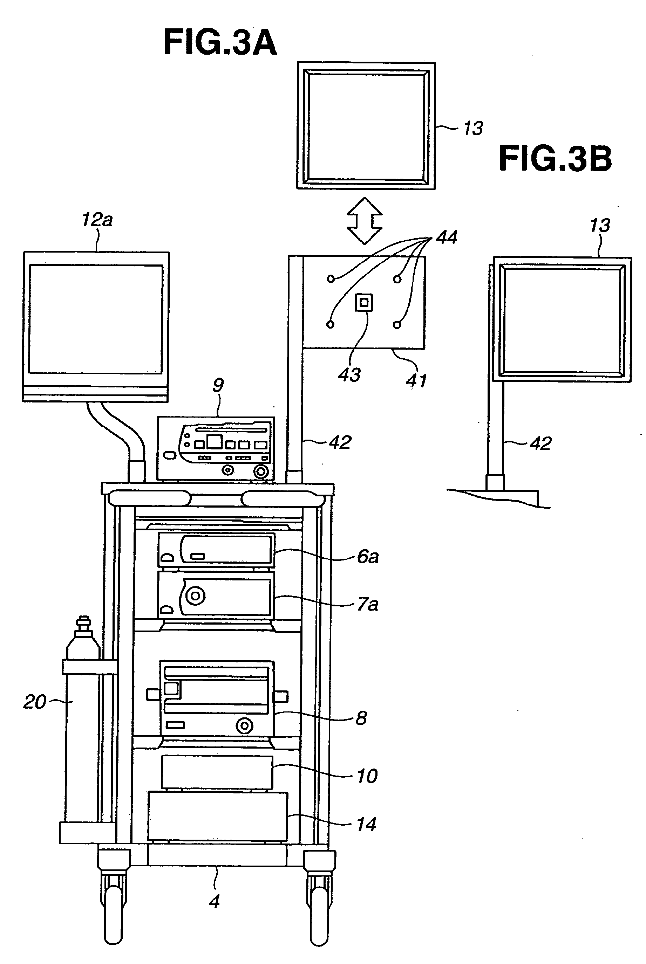

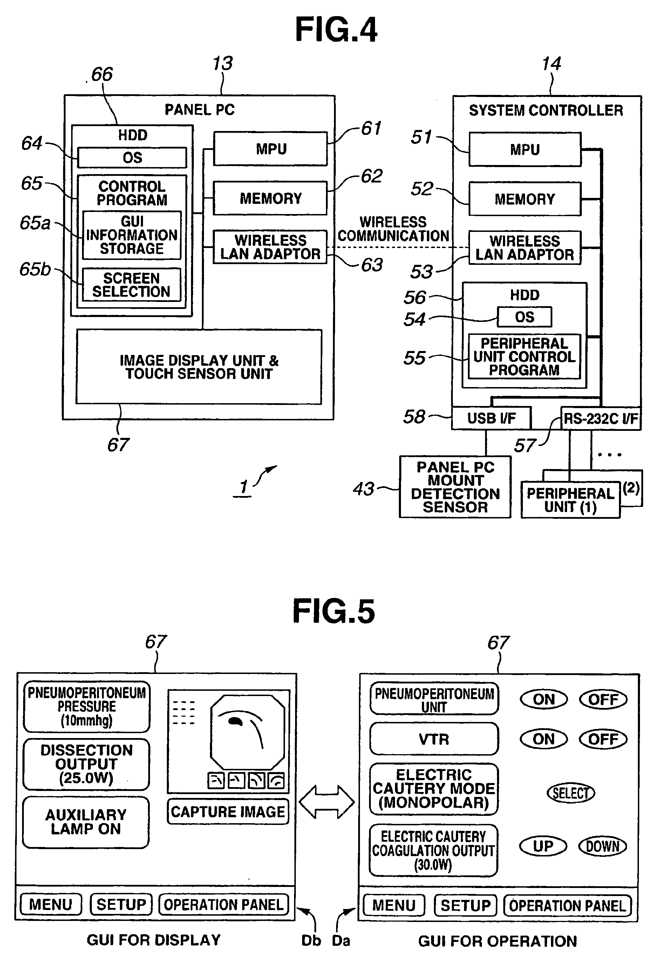

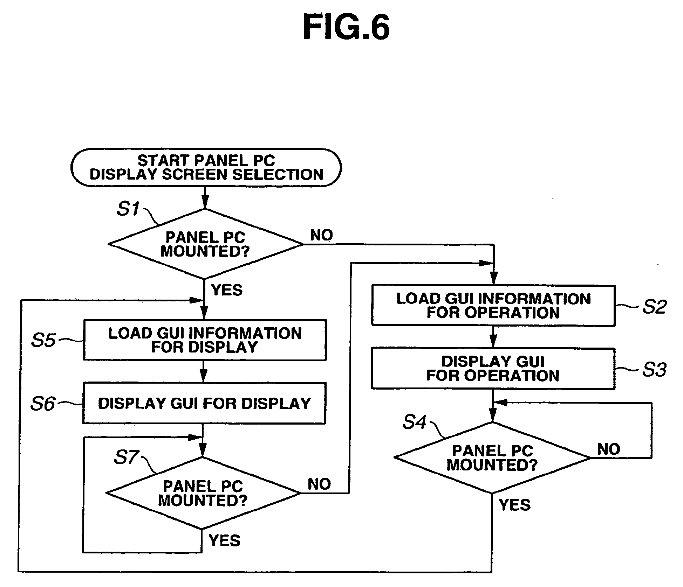

Referring to Figs. 1 to 6 with respect to Embodiment 1 of the present invention, Fig. 1 shows the general structure of the medical endoscope system according to the first embodiment of the present invention. Fig. 2 is a block diagram showing the main components of the structure shown in Fig. 1. Fig. 3 is a view showing the trolley to which the panel PC is detachably attached. Fig. 4 is a view showing the system controller and the panel PC including the inner structures. Fig. 5 is an example of the image displayed on the panel PC which displays the GUI for display and the GUI for operation which are selected in accordance with the detection result of the sensor. Fig. 6 is a flowchart showing the operation of the display screen selection control.

Referring to Fig. 1, the medical system according to the present invention, specifically, the medical endoscope system 1 includes a first trolley 4 and a second trolley 5 placed at both sides of the operating bed 2 on which a patient 3 lies. Each of the first and the second trolleys 4 and 5 includes a plurality of medical devices (peripheral devices of the endoscope) for observing, inspecting, treating and recording operations.

In the embodiment, the first trolley 4 includes a TV camera unit (or camera control unit) 6a, a light source unit 7a, a high-frequency cauterization unit (hereinafter referred to as an electric cautery unit) 8, a pneumoperitoneum unit 9, a VTR 10, a first endoscope monitor 12a, a panel type computer (hereinafter referred to as the panel PC) 13 which functions as the central display panel for displaying each state of the plurality of the medical devices and as the central operation panel for centrally performing the input operations to the plurality of the medical devices, a system controller 14 as a central control unit for centrally controlling the aforementioned medical devices.

The respective medical devices except the panel PC 13 are connected to the system controller 14 via not shown cables so as to allow the bi-directional communication therebetween.

The light source unit 7a is connected to an endoscope 16a via a light guide cable 15a for transmitting the illuminating light of the light source unit 7a to be applied to the (light guide of) the endoscope 16a. The site to be treated within the abdomen of the patient 3 into which the insertion portion of the endoscope 16a has been inserted is illuminated.

A camera head 18a equipped with an image pickup device is attached to an eye piece of the endoscope 16a. The optical image of the site to be treated or the like shot by the observation optical system of the endoscope 16a is picked up by the image pickup device within the camera head 18a, and transmitted to the TV camera unit 6a via a camera cable 19a so as to be processed into the signal by a signal processing circuit within the TV camera unit 6a. Then the video signal is generated and output to an endoscope monitor 12a for displaying the endoscope image of the site to be treated.

The pneumoperitoneum unit 9 is connected to a carbon dioxide bottle (hereinafter referred to as a compressed gas cylinder) 20, for example, such that the carbon dioxide gas is supplied into the abdominal cavity of the patient 3 through a pneumoperitoneum tube 21 extending from the pneumoperitoneum unit 9 connected to the compressed gas cylinder to the patient 3. In this case, the pneumoperitoneum tube 21 supplies the carbon dioxide gas into the abdominal cavity of the patient 3 through a guide tube 22 inserted into the abdomen of the patient 3.

The electric cautery unit 8 is connected to a treatment instrument 23 which is inserted into the abdominal cavity via the guide tube 22 through the cable 24, and further to a patient plate 25 which abuts on a wide area of the back of the patient 3 via a return cable 26.

The endoscope 16a is held by an endoscope holder 27 attached to the side surface of the operation bed 2.

The system controller 14 is connected to a microphone set 28 worn by the operator such that the voice command is input to the system controller 14.

The second trolley 5 includes such medical devices as an endoscope TV camera unit 6b, a light source unit 7b, an ultrasonic coagulation dissection unit 31, a video printer 32, a second endoscope monitor 12b, and a relay unit 33 and the like. The respective units are connected to the relay unit 33 via not shown cables to allow the bi-directional communication.

The light source unit 7b is connected to the endoscope 16b via a light guide cable 15b for transmitting the illuminating light from the light source unit 7b to be supplied to the (light guide of) the endoscope 16b so as to illuminate the site to be treated within the abdominal cavity of the patient 3 through which the insertion portion of the endoscope 16b has been inserted.

A camera head 18b equipped with an image pickup device is attached to an eye piece of the endoscope 16b. The optical image of the site to be treated shot by the observation optical system of the endoscope 16a is picked up by the image pickup device within the camera head 18b, and transmitted to a TV camera unit 6b via a camera cable 19a so as to be processed into the signal by a signal processing circuit within the TV camera unit 6b. Then the video signal is generated and output to an endoscope monitor 12b for displaying the endoscope view of the affected site.

The ultrasonic coagulation dissection unit 31 is connected to the treatment instrument 23 via a cable 34. The ultrasonic coagulation dissection unit 31 drives the ultrasonic oscillator of the treatment instrument 23 to allow coagulation or dissection using the ultrasonic wave. The treatment instrument may be used for the treatment as the electric cautery based on the high-frequency signal from the electric cautery unit 8.

The relay unit 33 is connected to the system controller 14 and the cable 35 to allow the bi-directional communication.

A remote controller (hereinafter referred to as "remote") 36 is connected to the relay unit 33. The operator operates the remote 36 from the sterilized area to transmit the operation signal to the system controller 14 via the relay unit 33. In response to the operation, the system controller 14 is allowed to execute the remote control of various units.

The trolley 4 is provided with a panel PC attachment mount 41 which allows detachable attachment of the panel PC 13.

Fig. 2 is a block diagram showing the structure of the system controller 14 and devices connected thereto in the medical endoscope system 1 as shown in Fig. 1. The TV camera units 6a, 6b, the light source units 7a, 7b, the electric cautery unit 8, the pneumoperitoneum unit 9, the VTR 10, the endoscope monitors 12a, 12b, the microphone set 28 for voice inputting, the ultrasonic coagulation dissection unit 31, and the remote 36 are connected to the system controller 14 via a serial interface of RS232C, for example, or the relay unit 33 so as to be controlled.

The TV camera units 6a, 6b, the VTR 10, the endoscope monitors 12a, 12b are connected to the system controller 14 via a not shown video cable or the relay unit 33 such that the video signal generated by the TV camera units 6a, 6b are output to the VTR 10 and the endoscope monitors 12a, 12b, respectively.

Referring to Fig. 3A, the flat panel PC attachment mount 41 is attached to the arm 42 which protrudes from the upper surface of the trolley 4. A panel PC mount detection sensor 43 is disposed at the center of the panel PC attachment mount 41, for example, to detect whether or not the panel PC 13 is set at the center of the panel PC attachment mount 41. Screw holes 44 for attachment of the panel PC are formed at a plurality of points around the sensor to fix the panel PC 13.

The panel PC attachment mount 41 is structured to allow the panel PC 13 shown thereabove to be detachably mounted. Fig. 3B at the right side of the drawing shows the state where the panel PC 13 is attached to the panel PC attachment mount 41. In Fig. 3B, the same components except the one adjacent to the panel PC attachment mount 41 shown in Fig. 3A will be omitted.

The panel PC mount detection sensor 43 is formed of a metal sensor which will be described later. Besides the metal sensor, a micro switch may be used to form the panel PC mount detection sensor which is turned ON from OFF when the back surface of the panel PC 13 is pressed upon its attachment to the panel PC attachment mount 41 so as to output the detection signal indicating whether or not the panel PC 13 has been attached to a predetermined position.

Fig. 4 is a view showing the structure of the system controller 14 and the periphery of the panel PC 13 in detail.

The system controller 14 includes an MPU 51 serving as a central processing unit (or micro processor) for central control processing of the entire system, a memory 52 used as the work area of the MPU 51 and the temporary data storage unit, a wireless LAN adaptor 53 which forms the wireless LAN, an operating system (hereinafter referred to as OS) 54, a hard disk (hereinafter referred to as HDD) 56 for storing the peripheral unit control program 55 and the like, an RS-232C interface (I/F) 57 for the bi-directional communication as the serial communication means with the peripheral units (1), (2), ..., and a USB I/F 58 for the bi-directional communication with the panel PC mount detection sensor 43 as the serial communication means, which are connected via bus.

The peripheral units (1), (2), ... represent the TV camera unit 6a and the like.

The panel PC 13 is formed of a panel type computer which allows the user to carry or grasp for easy operation. The panel PC 13 includes an MPU 61 which controls the respective units of the panel PC 13, a memory 62 used as the work area of the MPU 61 or for temporary storage of the data, a wireless LAN adaptor 63 which forms the wireless LAN, an operating system (hereinafter referred to as OS) 64, a hard disk (hereinafter referred to as HDD) 66 which stores the control program 65, and an image display unit & touch sensor unit 67 which serves to display the image and to detect the touch operation performed by the user, which are connected via the bus.

The image display unit & the touch sensor unit 67 includes a liquid crystal display as the image display unit (hereinafter referred to as LCD), and the touch sensor panel which detects the touch operation and touch operation position on the display of the LCD.

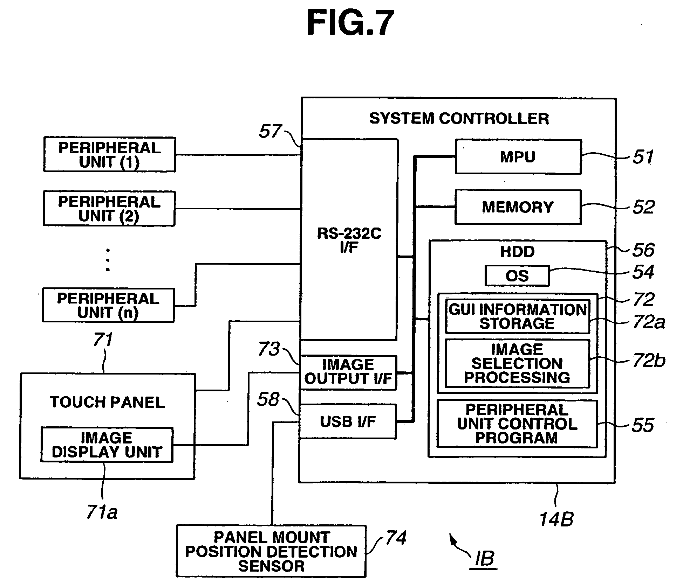

The control program 65 includes functions of a GUI information storage section (shown as GUI information storage in Fig. 4) 65a which stores graphical user interface (abbreviated as GUI) information for display and GUI information for operation, and a screen selection processing function (abbreviated as screen selection in Fig. 4 and also used in Fig. 7) 65b.

The screen selection processing function 65b loads one of the GUI information for display and the GUI information for operation which are stored in the GUI information storage section 65a to be displayed on the (image display unit of the) image display unit & touch sensor unit 67 such that the display screen is selected.

The MPU 61 executes the control operation in accordance with the control program 65 to load one of the GUI for display and the GUI for operation from the GUI information storage section 65a as the information memory unit, and to display the selected information on the (image display unit of) the image display unit & touch sensor unit 67 so as to select the display screen under the display screen selection control.

The information of the GUI for display and GUI for operation may be stored (recorded) in the memory 62 so as to execute the display screen selection control by selecting the address for loading the GUI information for display or the GUI information for operation to be displayed.

The MPU 61 of the panel PC 13 and the system controller 14 are allowed to perform the bi-directional communication via wireless LAN under the operation.

The MPU 61 of the panel PC 13 obtains the information with respect to the operation states of the peripheral units (1), (2), ... from the system controller 14, and stores the obtained information in the GUI information storage section 65a. The old information is updated by the new information. Accordingly, the MPU 61 includes the function as the information updating means for updating the GUI information.

When the GUI information for display is displayed, such information is the latest.

When the information of the GUI for display is displayed, in response to the change in the operation state of the peripheral units (1), (2), ..., the GUI information for display may be updated to the latest one under the control of the panel PC 13 or the system controller 14.

The system controller 14 transmits the control signal to the MPU 61 of the panel PC 13 to select the content to be displayed thereon in accordance with the detection result of the panel PC attachment by the panel PC mount detection sensor 42 via the wireless LAN.

The MPU 61 of the panel PC 13 executes the display screen selection control which selects the screen to be displayed on the (image display unit of the) image display unit & touch sensor unit 67 in accordance with the control signal.

In the aforementioned case, when the panel PC attachment is not detected, the system controller 14 allows the display content easily operated to be displayed on the operation panel. In the case where the panel PC attachment detection signal is detected, the control signal which allows the display content to be displayed on the display panel is transmitted to the MPU 61 of the panel PC 13.

The MPU 61 of the panel PC 13 changes the contents to be displayed on the image display unit & touch sensor unit 67 of the panel PC 13, and further changes the control content with respect to the touch operation.

More specifically, the MPU 61 selects the information to be displayed on the image display unit & touch sensor unit 67 (image display unit) between the GUI for display and the GUI for operation so as to be displayed as shown in Fig. 5 in accordance with the detection result of the panel PC mount detection signal.

In the present embodiment, the MPU 61 of the panel PC 13 serves to perform the display selection control function to change the display content on the image display unit & touch sensor unit 67 of the panel PC 13.

In the case where the panel PC 13 is not attached to a predetermined position where the panel PC mount detection sensor 43 is disposed, that is, the position of the panel PC attachment mount 41 in the embodiment, the GUI for operation is displayed as shown by Da in Fig. 5 at the right side. In the case where the panel PC 13 is attached to the panel PC attachment mount 41, the GUI for display is displayed as shown by Db in Fig. 5 at the left side. In other words, when the panel PC is removed from the predetermined position where the panel PC mount detection sensor 43 is disposed, the content displayed on the panel PC will be changed.

In the case of the display screen Da at the right side of Fig. 5, the nursing staff as the medical staff carries the panel PC 13 to display the peripheral units (specifically, the pneumoperitoneum unit, VTR) and the operation buttons for turning ON/OFF of the operation thereof, selector buttons for selecting the operation mode of the peripheral unit (specifically, the electric cautery), and output Up/Down button for changing the output of the peripheral unit for easy operation and setting of the peripheral units.

The screen displays buttons for menu which allows the user to select from a plurality of display contents, set up for setting, and display screen selection which allows manual selection of the GUI for display (as display panel).

Meanwhile on the display screen Db of the GUI for display at the left side which is displayed when the panel PC is mounted on the panel PC attachment mount 41, the set state of the peripheral unit, the output value, and the image captured in the image storage unit, for example, the VTR 10 are displayed. The operator is capable of recognizing the set state of the peripheral unit by observing the display.

The menu which allows the user to select from a plurality of display contents, set up for setting, and display screen selection button which allows the manual selection of the GUI for operation (as operation panel) are also displayed.

The medical endoscope system 1 according to the embodiment includes a function of the medical display unit for selecting the information between the GUI for operation and the GUI for display to be displayed on the image display unit & touch sensor unit 67 as the common panel unit.

The operation of the above structured system of the present embodiment will be described hereinafter.

In the case where the patient 3 receives the surgery under observation of the endoscope, the nursing staff mounts the peripheral units used for the surgery, for example, the TV camera unit 6a and the like on the trolleys 4 and 5 as shown in Fig. 1.

The endoscope 16a is connected to the TV camera unit 6a for the purpose of performing the inspection under observation of the endoscope. The treatment instrument 23 for treatment operations is also connected to the corresponding peripheral unit. Power is supplied to the medical endoscope system 1 to allow the nursing staff to set the peripheral unit to be used.

Upon power supply, the system controller 14 recognizes the connected peripheral units to become ready for executing the central control of the operation of the peripheral units. The system controller 14 is brought into the state to control the panel PC 13 via the wireless LAN.

In the aforementioned case, the system controller 14 monitors the detection signal of the panel PC mount detection sensor 43, and executes the display screen selection control on the image display unit & touch sensor unit 67 of the panel PC 13. Fig. 6 shows the selection control operation.

First in step S1, the MPU 51 of the system controller 14 monitors the detection signal of the panel PC mount detection sensor 43, and determines whether the panel PC 13 has been attached to the predetermined position of the panel PC mount detection sensor 43.

If it is determined that the panel PC has not been attached, the MPU 51 proceeds to execute step S2. If it is determined that the panel PC has been attached, the MPU 51 proceeds to execute step S5. In step S2, the MPU 51 wirelessly transmits the control signal to allow the MPU 61 of the panel PC 13 to display the GUI for operation.

In other words, the control signal corresponding to the detection result of the panel PC mount detection sensor 43 is wirelessly transmitted to the MPU 61 which actually executes the display selection control. In the aforementioned case, the MPU 51 may wirelessly transmit simply the signal of the detection result of the panel PC mount detection sensor 43.

The MPU 61 loads the GUI information for operation from the GUI information storage section 65a upon reception of the control signal.

In next step S3, the MPU 61 executes the control to display the GUI information for operation which has been loaded in step S2 by the image display unit & touch sensor unit 67. That is, the image display unit of the image display unit & touch sensor 67 displays as shown by the screen Da at the left side of Fig. 5.

The nursing staff operates the initial setting of the peripheral unit and the like while grasping the panel PC 13. In the aforementioned case, the panel PC 13 is structured to automatically display the screen Da of the GUI for operation through the aforementioned process, the operability is improved.

In step S4 subsequent to step S3, the MPU 51 of the system controller 14 monitors the detection signal of the panel PC mount detection sensor 43 (likewise step S1), and determines whether or not the panel PC 13 has been attached to the position of the panel PC mount detection sensor 43.

If it is determined that the panel PC 13 has not been attached, that is, the unattached state has been kept unchanged, the MPU 51 repeatedly executes step S4 while keeping the GUI for operation displayed. If it is determined that the panel PC 13 has been in the attached state (the unattached state has been changed to the attached state), the MPU 51 proceeds to execute step S5.

When the operation for initial setting of the peripheral unit is finished, the nursing staff attaches the panel PC 13 which has been used for setting to the panel PC attachment mount 41. Then the attachment of the panel PC 13 is detected by the panel PC mount detection sensor 43 so as to transmit the detection signal to the MPU 51. The MPU 51 then executes step S5.

In step S5, the MPU 51 wirelessly transmits the control signal which allows the MPU 61 of the panel PC 13 to display the GUI for display in accordance with the detection signal from the panel PC mount detection sensor 43.

In response to reception of the control signal, the MPU 61 loads the GUI information for display from the GUI information storage section 65a. Then in next step S6, the MPU 61 executes the control to display the GUI information for display on the image display unit & touch sensor unit 67. That is, the image display unit of the image display unit & touch sensor unit 67 displays the screen Db of the GUI for display shown at the left side of Fig. 5. Based on this, the operator is able to recognize the set state of the peripheral unit.

When the panel PC 13 is attached to the panel PC attachment mount 41, the display screen Db of the GUI for display is automatically selected. The medical staff does not have to perform the selection operation, resulting in improved operability.

In step S7 subsequent to step S6, the MPU 51 of the system controller 14 monitors the detection signal of the panel PC mount detection sensor 43 (likewise step S1), and determines whether the panel PC 13 has been attached to the position of the panel PC mount detection sensor 43.

When the unattached state is determined, that is, it is determined that the panel PC has been removed, the MPU 51 returns to step S2. Meanwhile, when the attached state is determined, execution of step S7 is continued.

In the thus operated embodiment, a single panel PC 13 may be used by selecting the function between the display panel and the operation panel. The determination is made whether or not the panel PC 13 is disposed at the predetermined position suitable for the display as the display panel or whether or not it is moved away from the predetermined position. Based on the determination result, the display is selected between the GUI for display and the GUI for operation, which makes it possible to secure the operability in the limited set space.

As the single panel PC 13 is used, the cost may be reduced compared to the case where two panel PCs are used. More space may also be ensured compared to the case where both the display panel and the operation panel are disposed, which releases the operator from feeling the narrowness.

### Embodiment 2

Embodiment 2 of the present invention will be described referring to Figs. 7 to 10. In Embodiment 1, the panel PC 13 is structured to select the content of the display screen. In the present embodiment, a touch panel 71 with the structure corresponding to that of the image display unit & touch sensor unit 67 in Embodiment 1 is used.

The present embodiment is structured to use a system controller 14B having a structure partially different from that of the system controller 14, and a touch panel 71 in place of the panel PC 13 of the medical endoscope system 1 shown in Fig. 1.

Fig. 7 is a view showing the system controller 14B and the touch panel 71 including the inner structures of a medical endoscope system 1B according to the present embodiment.

The system controller 14B of the present embodiment further includes a touch panel control program 72 in the system controller 14 shown in Fig. 4, for example, in the hard disk 56. The touch panel control program 72 includes a GUI information storage section 72a which contains the GUI for display and the GUI for operation, and a screen selection function 72b that changes the display screen on the touch panel 71.

In the system controller 14B according to the present embodiment, the image display unit 71 a of the touch panel 71 is provided with an image output I/F 73 for outputting the information with respect to the GUI for display and the GUI for operation. An RS-232C I/F 57 in the system controller 14B is connected to the touch panel 71 as well as to the peripheral units (1), (2), ... and (n). Upon operation of (the touch sensor portion of) the touch panel 71, the resultant operation signal is transmitted to the MPU 51. The MPU 51 then executes the corresponding control operation.

A USB I/F 58 is connected to a panel mount position detection sensor 74. The detection signal detected by the panel mount position detection sensor 74 is inputted to the MPU 51. The MPU 51 executes the control for selecting the display contents on the image display unit 71a of the touch panel 71 in accordance with the detection signal.

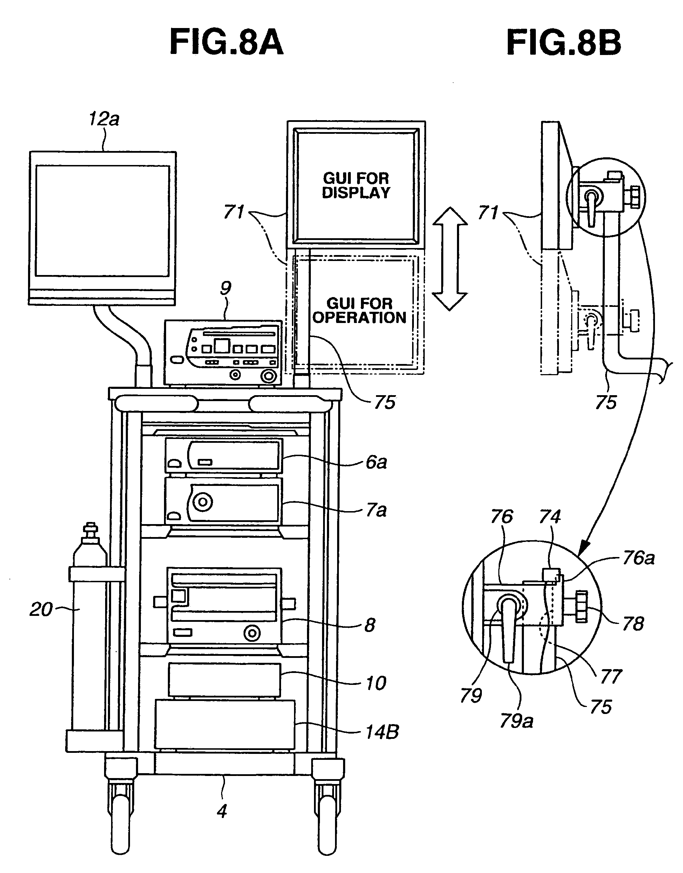

The touch panel 71 according to the present embodiment is structured to be slidably fixed along an arm 75 which protrudes upward from the trolley 4 as shown in Figs. 8A and 8B. Fig. 8A is a front view of the trolley 4 when viewed from the front. Fig. 8B is a side view of the trolley 4 when viewed from the side.

Referring to Fig. 8B and the enlarged view, an attachment member 76 of the touch panel 71 at the back side includes a hole 77 through which the arm 75 is slidably inserted and a fixing screw 78 such that the touch panel 71 is fixed at the arbitrary height position of the arm 75. The attachment member 76 includes a tilt mechanism 79 to tilt the display surface of the touch panel 71 by operating a knob 79a of the tilt mechanism 79.

In the example, the panel mount position detection sensor 74 is fixed at a position of the arm 75 at the height suitable for the display as the display panel to the upper tip of the arm 42 in this example. The panel mount position detection sensor 74 is formed of, for example, a metal sensor which detects the existence of a metal detection piece 76a attached to the attachment member 76 to protrude therefrom as shown in the enlarged view such that the determination is made whether the touch panel 71 is positioned at the predetermined height for the use as the display panel.

When the touch panel 71 is set at the position shown by the solid line in Figs. 8A and 8B, the MPU 51 of the system controller 14B executes the control for displaying the GUI for display in accordance with the detection signal of the panel mount position detection sensor 74. In the case where the detection signal is not outputted when the touch panel 71 moves down as shown by the chain double-dashed line, the MPU 51 of the system controller 14B executes the control for displaying the GUI for operation.

In the embodiment, only the single panel mount position detection sensor 74 is used for detecting whether or not the touch sensor 71 is positioned at the height for the use as the display panel. A second panel mount position detection sensor may be further provided at the height suitable for the operation as the operation panel, specifically, at the height lower than the upper tip of the arm 75 such that the display screen may be selected in accordance with the detection signals of the respective sensors.

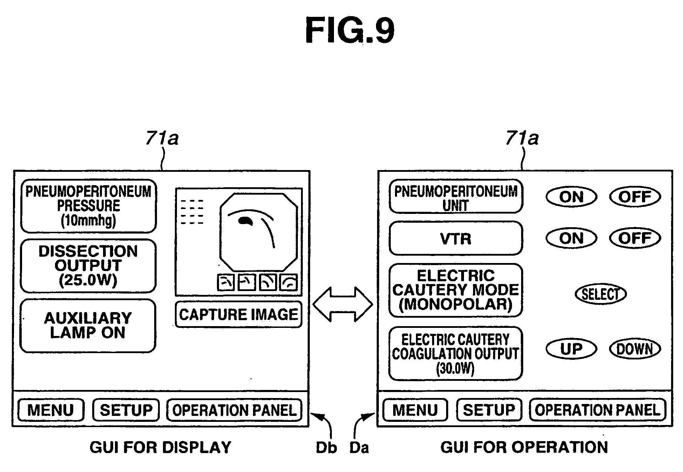

Fig. 9 shows the example of GUI for display and GUI for operation which are displayed on the image display unit 71 a of the touch panel 71 according to the embodiment. The example shown in Fig. 9 corresponds to the one shown in Fig. 5, for example.

In Embodiment 1, the control to select the information to be displayed on the panel PC 13 between the GUI for display and the GUI for operation is executed in accordance with the control signal of the system controller 14. In the present embodiment, the system controller 14B executes the control to select the information to be displayed on the image display unit 71 a of the touch panel 71 between the GUI for display and the GUI for operation.

The other structure is the same as that of Embodiment 1.

The control operation of the screen selection in the embodiment will be described.

Upon start of the operation of the display screen selection control of the touch panel, first in step S11, the MPU 51 determines whether or not the touch panel 71 has been disposed at the predetermined position for the display in accordance with the detection signal of the panel mount position detection sensor 74.

If the touch panel 71 has not been disposed at the position for the display, the MPU 51 loads the GUI information for operation from the GUI information storage section 72a in subsequent step S12. In next step S 13, the MPU 51 displays the GUI for operation on the image display unit 71 a of the touch panel 71.

Thereafter in next step S 14, the MPU 51 determines whether or not the touch panel 71 has been disposed at the position for the display in accordance with the detection signal of the panel mount position detection sensor 74 likewise the determination executed in step S11. If the touch panel 71 has not been disposed at the position for the display, the process in step S 14 is repeatedly executed.

Meanwhile, if it is determined that the touch panel 71 has been disposed at the position for the display, the process proceeds to step S 15.

In step S 15, the MPU 51 loads the GUI information for display from the GUI information storage section 72a. Next in step S 16, the MPU 51 displays the GUI for display on the image display unit 71 a of the touch panel 71. In next step S 17, the MPU 51 determines whether the touch panel 71 has been disposed at the position for the display based on the detection signal of the panel mount position detection sensor 74 likewise the determination executed in step S11.

If the touch panel 71 has been disposed at the position for the display, the process in step S17 is repeatedly executed.

Meanwhile, if it is determined that the touch panel 71 has not been disposed at the position for the display, the process returns to step S12.

In the embodiment for performing the aforementioned operation, the common touch panel 71 selects the function between the display as the display panel and display/operation as the operation panel likewise Embodiment 1. The cost may be reduced, and the system may be used in the narrow set space.

The touch panel 71 serves to be operated in place of the panel PC 13 in Embodiment 1, thus further reducing the cost. The embodiment provides substantially the same effects as those of Embodiment 1.

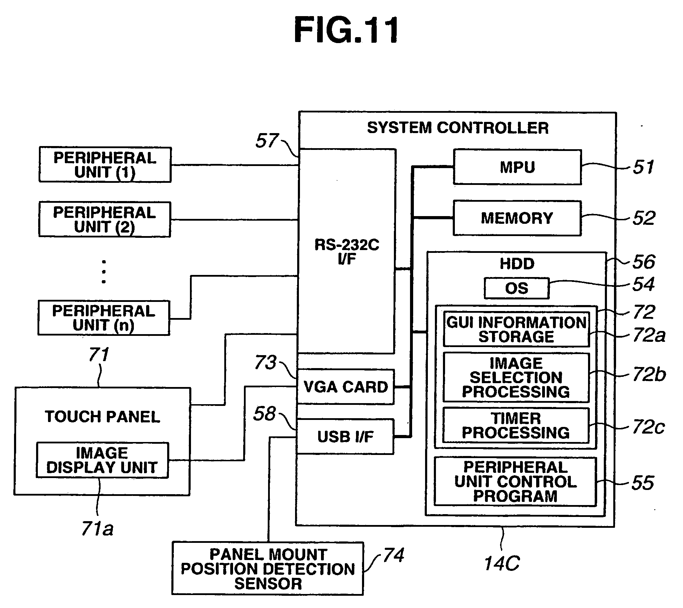

Next, a modified example of the present embodiment will be described. Fig. 11 shows the structure of a system controller 14C and the touch panel 71 of the modified example.

In the modified example, a timer process function 72c is added to the touch panel control program 72 in the hard disk 56 of the system controller 14B of Embodiment 2 such that the timer process function 72c executes the control to select the display screen if the panel mount position detection sensor 74 selects to disable the function for selecting the screen.

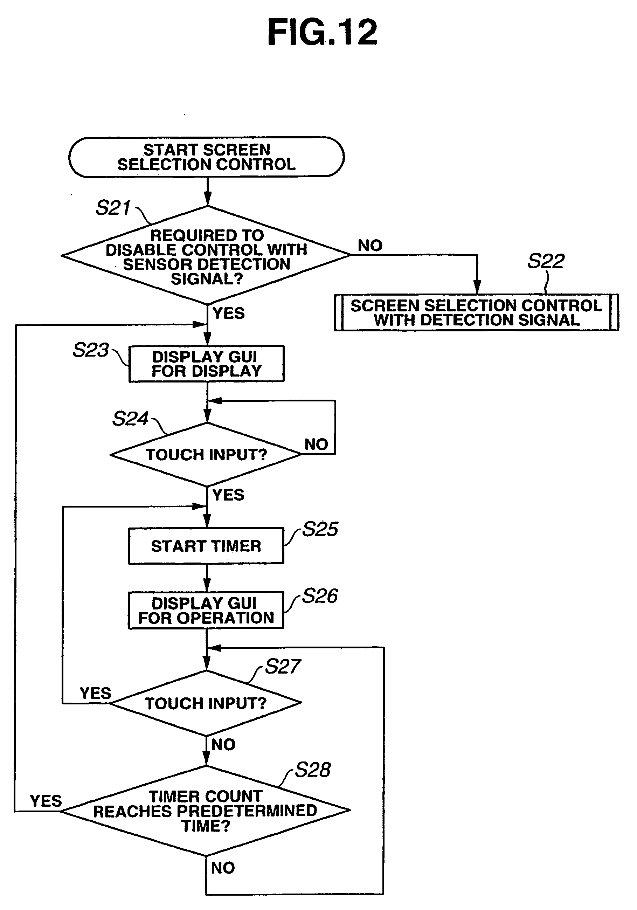

Other structures are the same as those of Embodiment 2. The operation of the modified example will be described referring to Fig. 12.

When the screen selection control starts, first in step S21, the MPU 51 determines whether or not the function for controlling the screen selection is disabled in accordance with the detection signal of the panel mount position detection sensor 74. The selection may be made in accordance with the convenience for the user, for example, the operator.

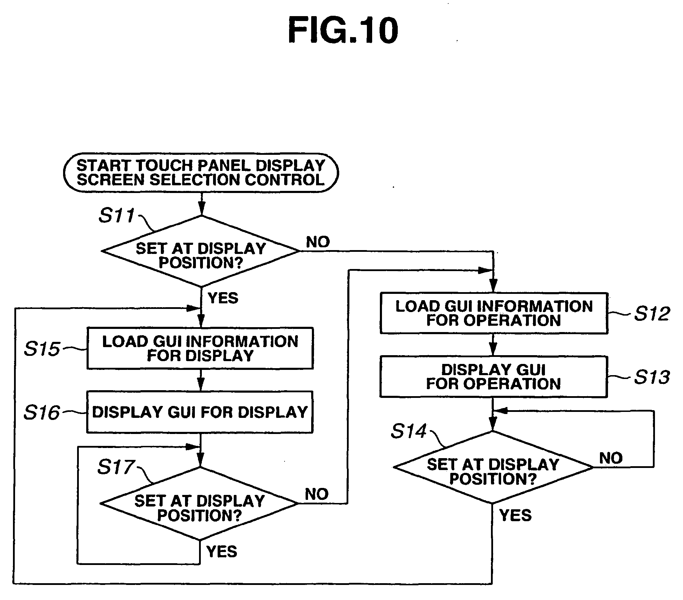

In the case where the screen selection control is executed based on the detection signal of the panel mount position detection sensor 74, the process proceeds to step S22. In the case where the screen selection control is disabled in accordance with the detection signal, the process proceeds to step S23. In step S22, steps from S11 to S16 are executed as described referring to Fig. 10. The explanation with respect to step S22, thus, will be omitted.

Meanwhile, in step S23, the MPU 51 loads the GUI information for display from the GUI information stored in the hard disk 56, and displays the GUI for display on the image display unit 71a of the touch panel 71. Next in step S24, the MPU 51 determines whether or not the touch input from the touch panel 71 exists. If the touch input does not exist, the aforementioned process is continued. If it is determined that the touch input exists, the MPU 51 starts the time count of the timer by the timer processing function 72c.

The MPU 25 loads the GUI information for operation from the GUI information stored in the hard disk 56 as shown in step S26 so as to be displayed on the image display unit 71a of the touch panel 71.

In next step S27, the MPU 51 determines whether or not the touch input from the touch panel 71 exists. If the touch input exists, the process returns to step S25 where the time count of the timer is started. In the case where the time count has been started before the aforementioned timing, the MPU 51 resets the timer and starts the time count.

In step S27, if it is determined that the touch input does not exist, the MPU 51 proceeds to step S28 where it is determined whether the time counted by the timer has reached the predetermined time.

If the counted time has not reached the predetermined time, the process returns to step S27 to be executed while keeping the display screen of the GUI for operation. Meanwhile, if the counted time has reached the predetermined time, the process returns to step S23 where the GUI for display is displayed on the image display unit 71a of the touch panel 71.

In the modified example, in the case of surgery, choices for the operator to control the display screen selection by the touch panel 71 may be widened, thus improving the operability and usability. The modified example provides substantially the same effects as those of Embodiment 2.

### Embodiment 3

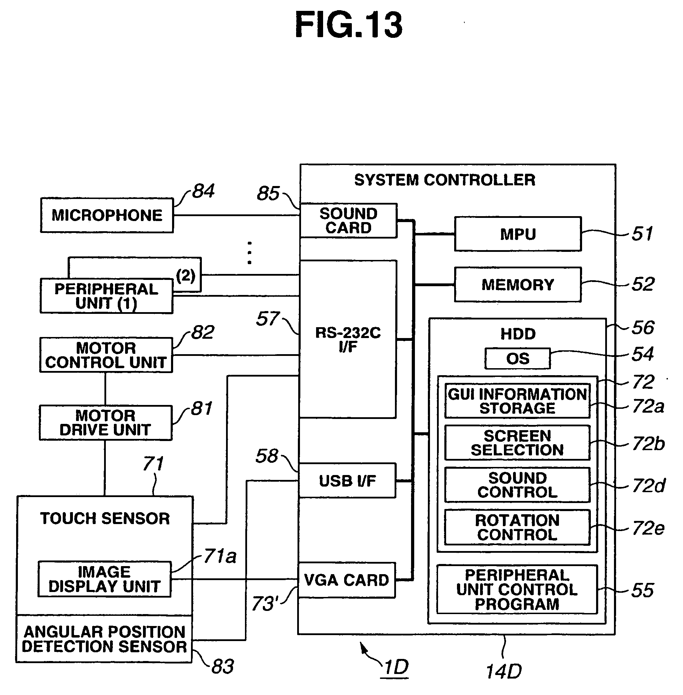

Embodiment 3 according to the present invention will be described referring to Figs. 13 to 15. In the embodiment, the direction of the display plane of the touch panel 71 is detected to execute the control for selecting the display screen. The structure of the system controller 14D and the periphery of the touch panel 71 in the medical endoscope system 1D according to the embodiment is shown in Fig. 13.

Referring to Fig. 13, the touch panel 71 includes a motor drive unit 81 that drives to rotate the touch panel 71, and a motor control unit 82 to control (a motor 81a of) the motor drive unit 81.

The touch panel 71 is provided with an angular position detection sensor 83 which detects a display direction of the display screen based on detection signals at a plurality of angular positions.

In the embodiment, a microphone 84 which receives the voice input from the operator is provided, and the system controller 14D is provided with a sound card 85 which executes the signal processing corresponding to the voice inputted through the microphone 84.

In the system controller 14D, the touch panel control program 72 within the hard disk 56 contains an audio control function 72d for driving the motor 81a of the motor drive unit 81 to be rotated and a rotation control function 72e to control the rotation of the motor 81 a through the audio control function 72d based on the sound subjected to the signal processing in the sound card 85.

In the embodiment, the GUI information storage section 72a contains GUI information for anesthesia as the GUI information suitable for the anesthetist who anaesthetizes to observe in addition to the information of the aforementioned GUI for display and the GUI for operation.

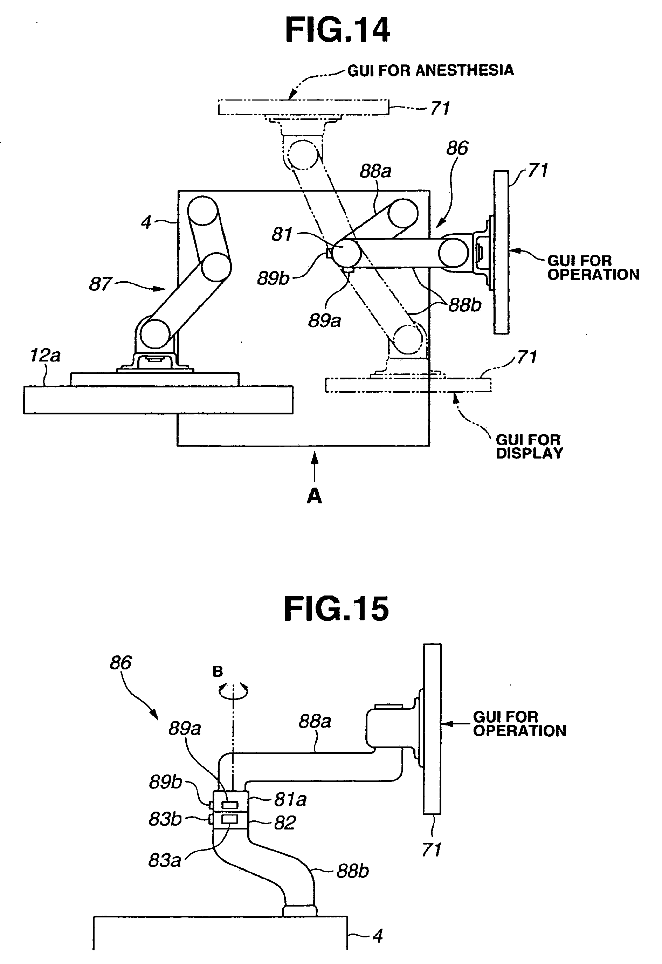

The motor control unit 82 is connected to the RS-232C I/F 57. Fig. 14 shows a touch panel holding mechanism 86 disposed on the upper surface of the trolley 4.

On the upper surface of the trolley 4, an arm mechanism 87 for holding the endoscope monitor 12a, and a touch panel holding mechanism 86 for holding the touch panel 71 which allows the display direction of the touch panel 71 to be variable.

The touch panel holding mechanism 86 includes a first arm 88a having its one end connected to the upper surface of the trolley 4, and a second arm 88b having one end rotatably connected to the other end of the first arm 88a and having the other end attached to the touch panel 71. A motor 81 a that forms the motor drive unit 81 is provided at the point where the first arm 88a is joined with the second arm.

When the motor 81a is driven to rotate, the second arm 88b rotates around the other end of the first arm 88a as shown in Fig. 14 so as to change the direction of the display plane of the touch panel 71.

Two first and second sensors 83a and 83b which form the angular position detection sensor 83 shown in Fig. 13 and two detection pieces 89a and 89b detected by the first and the second sensors 83a and 83b are provided at two positions around the portion where the first arm 88a is joined with the second arm 88b in the rotating direction. Both sensors 83a and 83b are formed of the metal sensors each detecting each of the metal detection pieces 89a and 89b, respectively.

Fig. 15 is a side view schematically showing the touch panel holding mechanism 86 when viewed from the arrow direction A shown in Fig. 14. The operation bed is provided opposite (lower section of the drawing) the arrow direction A shown in Fig. 14.

In the case where the operator who stands at the operation bed side easily identifies the display of the touch panel 71 opposite the arrow direction A, that is, the state shown by the chain double-dashed line in Fig. 14. If it is detected that the display plane of the touch panel 71 is directed to be in the aforementioned direction, the MPU 51 controls to display the GUI for display. In most cases, the nursing staff stands to the right side of the trolley 4 shown in Fig. 14 to set the peripheral units. In the case where it is detected that the display plane of the touch panel 71 is directed to the right side as shown by the solid line of Fig. 14, the control is executed to display the GUI for operation.

In most cases, the anesthetist performs the anesthesia treatment while observing the set state of the peripheral units at the position above the trolley 4 as shown in Fig. 14. If it is detected that the display plane of the touch panel 71 is directed opposite the aforementioned direction as shown by the chain double-dashed line of Fig. 14, the control is executed to display the GUI for anesthesia.

Referring to Fig. 15, the portion at which the first arm 88a is joined with the second arm 88b is provided with a motor control unit 82 fixed to the first arm 88a, and first and second sensors 83a, 83b at two positions in the circumferential direction to which the stator portion of the motor drive unit 81 is fixed. The detection pieces 89a, 89b are attached to the two positions in the circumferential direction of (a rotor of) the motor 81a, which are detected by the first and the second sensors 83a, 83b, respectively.

Referring to Fig. 15, in the state where the first sensor 83a and the second sensor 83b are disposed opposite the detection pieces 89a and 89b adjacent thereto, respectively, the sensors 83a, 83b output the respective detection signals to the MPU 51. In the aforementioned case, the MPU 51 executes the control to display the GUI for operation on the display of the touch panel 71.

In the state shown in Fig. 15, when the motor 81a is rotated in the direction of code B, only the second sensor 83b detects the detection piece 89a. In this case, the MPU 51 executes the control to display the GUI for display on the display of the touch panel 71.

In the state shown in Fig. 15, when the motor 81a is rotated in the inverse direction to the code B, for example, the first sensor 83a detects the detection piece 89b only. In the aforementioned case, the MPU 51 executes the control to display the GUI for anesthesia on the display of the touch panel 71.

In the embodiment, the rotation of the motor 81 a may be controlled through the voice inputted from the microphone 84.

The operation for selecting the display screen according to the above-structured embodiment will be described.

When the operation starts, first in step S31, the MPU 51 determines whether or not the voice inputted from the microphone 84 through the sound card 85 exists. If it is determined that the voice input exists, the MPU 51 allows the audio control function 72d to recognize the voice with respect to the command as to which direction the rotation is directed, or the command to stop the rotation in the rotating state.

Based on the voice recognition results, the MPU 51 allows the rotation control function 72e to drive the motor 81 a to rotate in the forward or the reverse direction, or to stop the rotation if the motor has been already rotating in step S33. The process then proceeds to step S34.

Meanwhile, in step S31, if it is determined that the voice input does not exist, the process proceeds to step S34. The MPU 51 determines whether or not detection is performed by the first sensor 83a and the second sensor 83b in step S34.

If the detection signals of both sensors 83a, 83b exist, the MPU 51 executes the control to display the GUI for operation on the display of the touch panel 71 as shown in step S35. The process then returns to step S31.

Meanwhile, if the detection signals from both sensors 83a, 83b do not exist in step S34, the MPU 51 determines whether or not the detection is made only by the first sensor 83a. If it is determined that the detection is made only by the first sensor 83a, the MPU 51 executes the control to display the GUI for anesthesia on the display of the touch panel 71 in step S37. The process then returns to step S31.

Meanwhile, if it is determined that no detection signal only of the first sensor 83a exists in step S36, the MPU 51 determines that the second sensor 83b is only in the detection state in step S38, and executes the control to display the GUI for display on the display of the touch panel 71. Then the process returns to step S31 where the aforementioned process is repeatedly executed.

In the above-operated embodiment, in the case where the touch panel 71 is controlled to be rotated by the motor 81a based on the voice, and the touch panel 71 is set to the predetermined rotational angular position, the sensors 83a, 83b detect the rotational angular positions such that the display screen is selected suitable for the detected rotational angular position. This may reduce the cost, allow the use in the narrow set space, and improve the operability to the great degree.

In the embodiment, the screen may be selected among three display screens, thus reducing the cost to the great degree.

In the case where the motor 81a is rotated to be positioned at the rotational angular position for displaying the GUI for display in the state where the GUI for operation has been displayed, the content to be displayed is changed to the GUI for display in accordance with the detection signal of the two sensors 83a, 83b. The rotation of the motor 81a may be stopped accompanied with the aforementioned operation.

In the aforementioned explanation, the display screen is selected through the voice input. However, the motor 81a may be rotated by switching operations, or the second arm 88b is manually rotated without the motor drive unit 81 and the like to select the display screen based on the detection signals of the first and the second sensors 83a and 83b. Alternatively, the structure for performing the rotation using the spring, which is easier compared to the manual operation, may be employed.

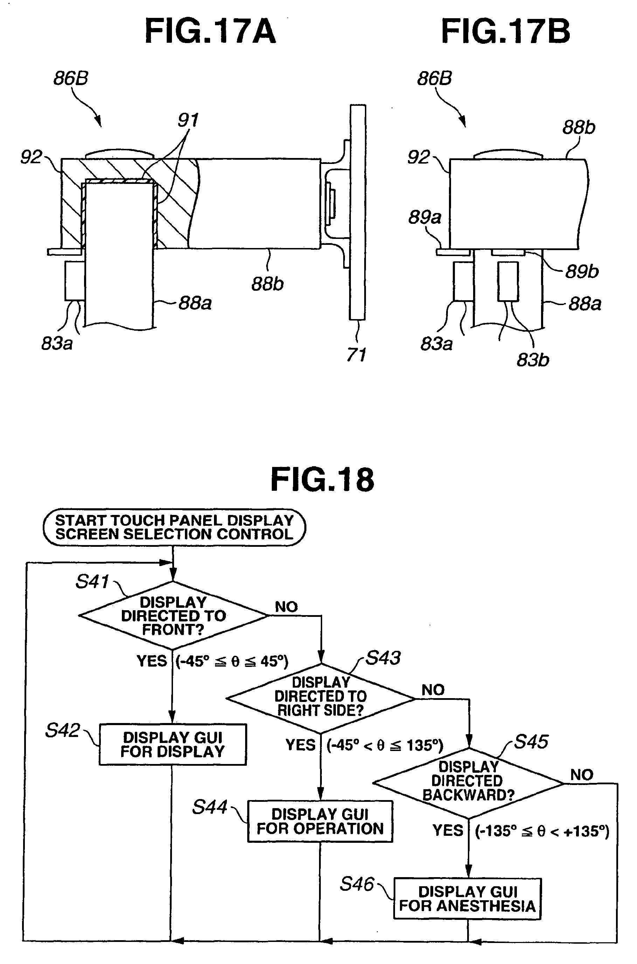

Figs. 17A and 17B show the structure of the portion around the joint section between the first arm 88a and the second arm 88b of the touch panel holding mechanism 86B according to the modified example. Fig. 17A is a sectional view showing the portion around the rear end of the second arm 88a, and Fig. 17B is a side view.

Referring to Fig. 17A, a rotating holding portion 92, in which the rear end (proximal end) of the second arm 88b is rotatably held around the center axis of the first arm 88a through a resin collar 91, is formed at the upper end of the first arm 88a. The rotating holding portion 92 may be structured to rotatably support the second arm 88b such that the second arm 88b is manually rotated (rotatably operated), or structured to drive for rotation using the motor through the operation of a switch.

Referring to Fig. 17B, the first sensor 83a and the second sensor 83b are fixed on the circumference of the first arm 88a arranged to form the angle of, for example, 90° with fixing screws. The detection pieces 89a and 89b are fixed with not shown fixing screws arranged to form the angle of, for example, 90° on the circumference at the rear end of the second arm 88b.

In the modified example, the respective sensors 83a, 83b detect whether or not the detection pieces exist within the range of the distance from the respective attachment positions of the sensors between -45° and +45°. The MPU 51 detects the position or direction of the display plane of the touch panel 71 which has been set (the center angle of the direction: θ) to execute the control to select the display screen. In Figs. 17A and 17B, the front side of the trolley 4 is defined as the front in the vertical direction of the drawing.

Fig. 18 shows the control to select the display of the touch panel 71. The explanation will be made on the assumption that the angle formed when the direction of the display plane of the touch panel 71 corresponds to the front of the trolley 4 is set to the value of 0.

When the operation starts, first in step S41, the MPU 51 determines whether the display plane of the touch panel 71 is directed to the front (that is, -45° ≤ θ ≤ + 45°) in accordance with the detection signals of the first and the second sensors 83a, 83b.

If it is determined that the display plane is directed to the front, the MPU 51 executes the control to display the GUI for display on the display of the touch panel 71 in next step S42, and returns to step S41.

Meanwhile, if it is determined that the display is not directed to the front in step S41, the MPU 51 determines whether the display of the touch panel 71 is directed to the right side (that is, + 45° < θ ≤ + 135°) in accordance with the detection signals of the first and the second sensors 83a, 83b.

If it is determined that the display plane is directed to the right side, the MPU 51 executes the control to display the GUI for operation on the display of the touch panel 71 in next step S44, and returns to step S41.

Meanwhile, if it is determined that the display plane is not directed to the right side in step S43, the MPU 51 determines whether the display plane of the touch panel 71 is directed backward (that is, -135° ≤ θ ≤ + 135°) in accordance with the detection signals of the first and the second sensors 83a, 83b.

If it is determined that the display is directed backward, the MPU 51 executes the control to display the GUI for anesthesia on the display of the touch panel in next step S46, and returns to step S41.

Meanwhile, if it is determined that the display plane is not directed backward in step S45, the process returns to step S41.

In the above operated modified example, the cost may be reduced, and the system may be used in the narrow set space.

In the embodiments and the modified examples thereof, the sensor means in the form of two sensors 83a, 83b is used to detect the direction (position) of the display plane of the touch panel 71 which is rotatably held. However, the rotary encoder may be attached to the rotatable shaft to detect the predetermined direction based on the resultant detection signals without being limited to those described above.

In the embodiments and the modified examples thereof, the panel PC 13 and the like may be used in place of the touch panel 71.

Note that embodiments and the like configured by partially combining the above-described embodiments also belong to the present invention.

### Industrial Applicability

When surgery and the like are performed under observation of the endoscope by using a plurality of peripheral units, the display screen selection control is executed to cause the display screen to selectively display the operation panel function and the display panel function, thereby reducing the cost and enabling the system to be used in a narrow set space.

## Claims

1. A medical system (1) comprising:
a plurality of medical devices (6a, 6b, 7a, 7b);
a medical display apparatus (13) including
a panel portion (67) including a display unit (67) configured to display information with respect to the plurality of medical devices (6a, 6b, 7a, 7b) and a sensor unit (67) configured to detect an input operation; and
an information storage unit (65a) configured to store graphical user interface information for display and graphical user interface information for operation; and
a detection unit (43) configured to output a detection signal which detects whether or not the panel portion (67) is disposed at least at one predetermined position,
**characterized in that**
the plurality of medical devices (6a, 6b, 7a, 7b) are a plurality of endoscope peripheral devices to be used together with an endoscope (16a),
the medical display apparatus (13) is configured to be operated by being grasped by a nursing staff member, wherein
the medical system (1) further comprises
a controller (14) configured to perform bi-directional communication with the plurality of endoscope peripheral devices and execute central control of the plurality of endoscope peripheral devices; and
a display selection control unit (61) provided in the medical display apparatus (13) and configured to select information between the graphical user interface information for display which is information for display regarding an operation state of the plurality of endoscope peripheral devices, and the graphical user interface information for operation for operating the plurality of endoscope peripheral devices stored in the information storage unit (65a), in accordance with a control signal wirelessly transmitted based on the detection signal of the detection unit (43) so as to be displayed on the display unit (67), wherein
the display selection control unit (61) is configured, on the basis of the control signal, to control to cause the display unit (67) to display the graphical user interface information for display when it is detected that the panel portion (67) is at the predetermined position, and control to cause the display unit (67) to display the graphical user interface information for operation when it is not detected that the panel portion (67) is at the predetermined position.

2. The medical system (1) according to Claim 1, wherein the panel portion (67) is formed of a touch panel (71) which includes a touch sensor unit provided to the display unit (67) as the sensor unit for detecting a touch operation, or a panel type computer (13) including the information storage unit (65a) in addition to the touch panel (71).

3. The medical system (1) according to Claim 1, wherein the detection unit (43) includes a sensor configured to perform a detection whether the panel portion (67) is detachably attached to the predetermined position, or a detection whether the panel portion (67) which is movable is set at the predetermined position.

4. The medical system (1) according to Claim 1, wherein:
the panel portion (67) is rotatably held by a rotating portion at an angle including a rotating angle at the predetermined position; and
the display selection control unit (61) is configured to execute the control in accordance with a detection signal of the detection unit.

5. The medical system (1) according to Claim 1, further comprising a timer unit (72c) configured to start time count in response to an input operation to the sensor unit, wherein the display selection control unit (61) is configured to select the information between the graphical user interface information for display and the graphical user interface information for operation based on a result of the time count performed by the timer unit (72c) so as to be displayed.

6. The medical system (1) according to Claim 1, wherein:
the detection unit (43) is configured to determine whether or not the panel portion (67) is disposed at three different predetermined positions; and
the display selection control unit (61) is configured to select the information among the graphical user interface information for display, the graphical user interface information for operation, and third graphical user interface information which is different from the graphical user interface information for display and the graphical user interface information for operation so as to be displayed.

7. The medical system (1) according to Claim 1, wherein the display selection control unit (61) is formed of a controller (14) which is configured to control the plurality of medical devices (6a, 6b, 7a, 7b).

8. The medical system (1) according to Claim 2, wherein when the panel portion (67) is formed of the panel type computer (13), the display selection control unit (61) is formed of a microprocessor (61) which is configured to select a display content on the display unit (67) in accordance with a detection signal of the detection unit (43).

9. The medical system (1) according to Claim 2, wherein the detection signal of the detection unit (43) is configured to be wirelessly transmitted to the display selection control unit (61).

10. The medical system (1) according to Claim 1 further comprising an information update unit (61) configured to update the graphical user interface information for display in accordance with operation states of the plurality of medical devices (6a, 6b, 7a, 7b).

## Patentansprüche

1. Medizinisches System (1), das umfasst:
eine Mehrzahl von medizinischen Einrichtungen (6a, 6b, 7a, 7b);
ein medizinisches Anzeigegerät (13), das umfasst:
einen Panelabschnitt (67) mit einer Anzeigeeinheit (67), die dazu eingerichtet ist, eine Information bezüglich der Mehrzahl von medizinischen Einrichtungen (6a, 6b, 7a, 7b) anzuzeigen, und einer Sensoreinheit (67), die dazu eingerichtet ist, eine Eingabebetätigung zu erfassen; und
eine Informationsspeichereinheit (65a), die dazu eingerichtet ist, eine grafische Benutzerschnittstelleninformation für eine Anzeige und eine grafische Benutzerschnittstelleninformation für eine Betätigung zu speichern; und
eine Erfassungseinheit (43), die dazu eingerichtet ist, ein Erfassungssignal auszugeben, das erfasst, ob der Panelabschnitt (67) an mindestens einer vorbestimmten Position angeordnet ist oder nicht,
**dadurch gekennzeichnet, dass**
die Mehrzahl von medizinischen Einrichtungen (6a, 6b, 7a, 7b) eine Mehrzahl von Endoskop-Peripherieeinrichtungen zur Nutzung gemeinsam mit einem Endoskop (16a) sind,
das medizinische Anzeigegerät (13) dazu eingerichtet ist, betätigt zu werden, indem es von einem Betreuungspersonalmitglied gegriffen wird, wobei
das medizinische System (1) ferner umfasst
eine Steuerung (14), die dazu eingerichtet ist, eine bidirektionale Kommunikation mit der Mehrzahl von Endoskop-Peripherieeinrichtungen durchzuführen und eine zentrale Steuerung der Mehrzahl von Endoskop-Peripherieeinrichtungen auszuführen; und
eine Anzeigeauswahlsteuereinheit (61), die in dem medizinischen Anzeigegerät (13) vorgesehen und dazu eingerichtet ist, eine Information zwischen der grafischen Benutzerschnittstelleninformation für eine Anzeige, die eine Information für eine Anzeige bezüglich eines Betätigungszustands der Mehrzahl von Endoskop-Peripherieeinrichtungen ist, und der grafischen Benutzerschnittstelleninformation für eine Betätigung für eine Betätigung der Mehrzahl von Endoskop-Peripherieeinrichtungen, die in der Informationsspeichereinheit (65a) gespeichert sind, gemäß einem Steuersignal auszuwählen, das auf der Basis des Erfassungssignals der Erfassungseinheit (43) drahtlos übertragen worden ist, um auf der Anzeigeeinheit (67) angezeigt zu werden, wobei
die Anzeigeauswahlsteuereinheit (61) dazu eingerichtet ist, auf der Basis des Steuersignals zu steuern, um die Anzeigeeinheit (67) zu veranlassen, die grafische Benutzerschnittstelleninformation für eine Anzeige anzuzeigen, wenn erfasst worden ist, dass sich der Panelabschnitt (67) an der vorbestimmten Position befindet, und zu steuern, um die Anzeigeeinheit (67) zu veranlassen, die grafische Benutzerschnittstelleninformation für eine Betätigung anzuzeigen, wenn nicht erfasst worden ist, dass sich der Panelabschnitt (67) an der vorbestimmten Position befindet.

2. Medizinisches System (1) nach Anspruch 1, bei dem der Panelabschnitt (67) aus einem berührungssensitiven Panel (71), das eine Berührungssensoreinheit umfasst, die an der Anzeigeeinheit (67) als die Sensoreinheit zur Erfassung einer Berührungsbetätigung vorgesehen ist, oder einem Paneltypcomputer (13), der zusätzlich zu dem berührungssensitiven Panel (71) die Informationsspeichereinheit (65a) umfasst, gebildet ist.

3. Medizinisches System (1) nach Anspruch 1, bei dem die Erfassungseinheit (43) einen Sensor umfasst, der dazu eingerichtet ist, eine Erfassung, ob der Panelabschnitt (67) lösbar an der vorbestimmten Position befestigt ist, oder eine Erfassung, ob der Panelabschnitt (67), der bewegbar ist, an der vorbestimmten Position angeordnet ist, durchzuführen

4. Medizinisches System (1) nach Anspruch 1, bei dem:
der Panelabschnitt (67) durch einen Drehabschnitt drehbar in einem Winkel gehalten wird, der einen Drehwinkel an der vorbestimmten Position umfasst; und
der Anzeigeauswahlsteuerabschnitt (61) dazu eingerichtet ist, die Steuerung gemäß einem Erfassungssignal der Erfassungseinheit auszuführen.

5. Medizinisches System (1) nach Anspruch 1, das ferner eine Zeitnehmereinheit (72c) umfasst, die dazu eingerichtet ist, in Reaktion auf eine Eingabebetätigung in die Sensoreinheit eine Zeitnehmung zu starten, wobei der Anzeigeauswahlsteuerabschnitt (61) dazu eingerichtet ist, die Information zwischen der grafischen Benutzerschnittstelleninformation für eine Anzeige und der grafischen Benutzerschnittstelleninformation für eine Betätigung auf der Basis eines Ergebnisses der von der Zeitnehmereinheit (72c) durchgeführten Zeitnehmung auszuwählen, um angezeigt zu werden.

6. Medizinisches System (1) nach Anspruch 1, bei dem:
die Erfassungseinheit (43) dazu eingerichtet ist, zu bestimmen, ob der Panelabschnitt (67) an drei verschiedenen vorbestimmten Positionen angeordnet ist oder nicht; und
die Anzeigeauswahlsteuereinheit (61) dazu eingerichtet ist, die Information aus der grafischen Benutzerschnittstelleninformation für eine Anzeige, der grafischen Benutzerschnittstelleninformation für eine Betätigung und einer dritten grafischen Benutzerschnittstelleninformation, die sich von der grafischen Benutzerschnittstelleninformation für eine Anzeige und der grafischen Benutzerschnittstelleninformation für eine Betätigung unterscheidet, auszuwählen, um angezeigt zu werden.

7. Medizinisches System (1) nach Anspruch 1, bei dem die Anzeigeauswahlsteuereinheit (61) durch eine Steuerung (14) gebildet ist, die dazu eingerichtet ist, die Mehrzahl von medizinischen Einrichtungen (6a, 6b, 7a, 7b) zu steuern.

8. Medizinisches System (1) nach Anspruch 2, bei dem, wenn der Panelabschnitt (67) aus dem Paneltypcomputer (13) gebildet ist, die Anzeigeauswahlsteuereinheit (61) aus einem Mikroprozessor (61) gebildet ist, der dazu eingerichtet ist, einen Anzeigeinhalt auf der Anzeigeeinheit (67) gemäß einem Erfassungssignal der Erfassungseinheit (43) auszuwählen.

9. Medizinisches System (1) nach Anspruch 2, bei dem das Erfassungssignal der Erfassungseinheit (43) dazu eingerichtet ist, drahtlos an die Anzeigeauswahlsteuereinheit (61) übertragen zu werden.

10. Medizinisches System (1) nach Anspruch 1, das ferner eine Informationsaktualisierungseinheit (61) umfasst, die dazu eingerichtet ist, die grafische Benutzerschnittstelleninformation für eine Anzeige gemäß Betätigungszuständen der Mehrzahl von medizinischen Einrichtungen (6a, 6b, 7a, 7b) zu aktualisieren.

## Revendications

1. Système médical (1) comprenant :
une pluralité de dispositifs médicaux (6a, 6b, 7a, 7b) ;
un appareil d'affichage médical (13) comprenant
une partie de panneau (67) comprenant une unité d'affichage (67) configurée pour afficher des informations par rapport à la pluralité de dispositifs médicaux (6a, 6b, 7a, 7b) et une unité de capteurs (67) configurée pour détecter une opération d'entrée ; et
une unité de stockage d'informations (65a) configurée pour stocker des informations d'interface utilisateur graphiques pour l'affichage et des informations d'interface utilisateur graphiques pour le fonctionnement ; et
une unité de détection (43) configurée pour émettre un signal de détection qui détecte si la partie de panneau (67) est au moins disposée ou non à une position prédéterminée,
**caractérisé en ce que**
la pluralité de dispositifs médicaux (6a, 6b, 7a, 7b) est une pluralité de dispositifs périphériques d'endoscopes destinés à être utilisés conjointement avec un endoscope (16a),
l'appareil d'affichage médical (13) est configuré pour être actionné en étant saisi par un membre du personnel infirmier, dans lequel
le système médical (1) comprend en outre
une commande (14) configurée pour effectuer une communication bidirectionnelle avec la pluralité de dispositifs périphériques d'endoscopes et effectuer une commande centrale de la pluralité de dispositifs périphériques d'endoscopes ; et
une unité de commande de sélection d'affichage (61) prévue dans l'appareil d'affichage médical (13) et configurée pour sélectionner des informations entre les informations d'interface utilisateur graphiques d'affichage qui sont des informations d'affichage concernant un état de fonctionnement de la pluralité de dispositifs périphériques d'endoscope, et les informations d'interface utilisateur graphiques de fonctionnement pour faire fonctionner la pluralité de dispositifs périphériques d'endoscope stockés dans l'unité de stockage d'informations (65a), selon un signal de commande transmis sans fil en se basant sur le signal de détection de l'unité de détection (43) de façon à être affichées sur l'unité d'affichage (67), dans lequel
l'unité de commande de sélection d'affichage (61) est configurée, en se basant sur le signal de commande, pour commander l'unité d'affichage (67) pour qu'elle affiche les informations d'interface utilisateur graphiques pour l'affichage lorsqu'il est détecté que la partie de panneau (67) est à une position prédéterminée, et commander l'unité d'affichage (67) pour qu'elle affiche les informations d'interface utilisateur graphiques pour le fonctionnement lorsqu'il n'est pas détecté que la partie de panneau (67) est à une position prédéterminée.

2. Système médical (1) selon la revendication 1, dans lequel la partie de panneau (67) est formée d'un panneau tactile (71) qui comprend une unité de capteurs tactiles prévue sur l'unité d'affichage (67) en tant que l'unité de capteurs pour détecter une opération tactile, ou un ordinateur de type panneau (13) comprenant l'unité de stockage d'informations (65a) en plus du panneau tactile (71).

3. Système médical (1) selon la revendication 1, dans lequel l'unité de détection (43) comprend un capteur configuré pour détecter si la partie de panneau (67) est fixée de façon amovible à la position prédéterminée ou détecter si la partie de panneau (67) qui est mobile est réglée à la position prédéterminée.

4. Système médical (1) selon la revendication 1, dans lequel :
la partie de panneau (67) est maintenue en rotation par une partie rotative à un angle comprenant un angle de rotation à la position prédéterminée ; et
l'unité de commande de sélection d'affichage (61) est configurée pour exécuter la commande selon un signal de détection de l'unité de détection.

5. Système médical (1) selon la revendication 1, comprenant en outre une unité d'horloge (72c) configurée pour démarrer un comptage temporel en réponse à une opération d'entrée vers l'unité de capteurs, dans lequel l'unité de commande de sélection d'affichage (61) est configurée pour sélectionner les informations entre les informations d'interface utilisateur graphiques pour l'affichage et les informations d'interface utilisateur graphiques pour le fonctionnement en se basant sur un résultat du comptage temporel effectué par l'unité d'horloge (72c) de façon à être affichées.

6. Système médical (1) selon la revendication 1, dans lequel :
l'unité de détection (43) est configurée pour déterminer si la partie de panneau (67) est disposée ou non sur trois positions prédéterminées différentes ; et
l'unité de commande de sélection d'affichage (61) est configurée pour sélectionner les informations parmi les informations d'interface utilisateur graphiques pour l'affichage, les informations d'interface utilisateur graphiques pour le fonctionnement, et des troisièmes informations d'interface utilisateur graphiques qui sont différentes des informations d'interface utilisateur graphiques pour l'affichage et des informations d'interface utilisateur graphiques pour le fonctionnement de façon à être affichées.

7. Système médical (1) selon la revendication 1, dans lequel l'unité de commande de sélection d'affichage (61) est formée d'une commande (14) qui est configurée pour commander la pluralité de dispositifs médicaux (6a, 6b, 7a, 7b).

8. Système médical (1) selon la revendication 2, dans lequel lorsque la partie de panneau (67) est formée de l'ordinateur de type panneau (13), l'unité de commande de sélection d'affichage (61) est formée d'un microprocesseur (61) qui est configuré pour sélectionner un contenu d'affichage sur l'unité d'affichage (67) selon un signal de détection de l'unité de détection (43).

9. Système médical (1) selon la revendication 2, dans lequel le signal de détection de l'unité de détection (43) est configuré pour être transmis sans fil à l'unité de commande de sélection d'affichage (61).

10. Système médical (1) selon la revendication 1, comprenant en outre une unité de mise à jour des informations (61) configurée pour mettre à jour les informations d'interface utilisateur graphiques pour l'affichage selon des états de fonctionnement de la pluralité de dispositifs médicaux (6a, 6b, 7a, 7b).
